# EUROPÄISCHE PATENTANMELDUNG

(11) **EP 0 781 771 A1**
(43) Veröffentlichungstag der Anmeldung: **02.07.1997**
(21) Anmeldenummer: 96116932.3
(22) Anmeldetag: 22.10.1996
(51) Int. Cl.: C07D 403/14, C08G 18/79, C09J 175/04

(54) **Cyclische Amidin- sowie Uretdiongruppen enthaltende Verbindungen, ein Verfahren zu ihrer Herstellung sowie deren Verwendung**

(30) Priorität: 28.12.1995 DE 19549029
(71) Anmelder: HÜLS AKTIENGESELLSCHAFT, 45764 Marl (DE)
(72) Erfinder: Gras, Rainer, Dr., 44879 Bochum (DE); Wolf, Elmar, Dr., 45661 Recklinghausen (DE)

(57) **Zusammenfassung**

Cyclische Amidin- sowie Uretdiongruppen enthaltende Verbindungen folgender Zusammensetzung: 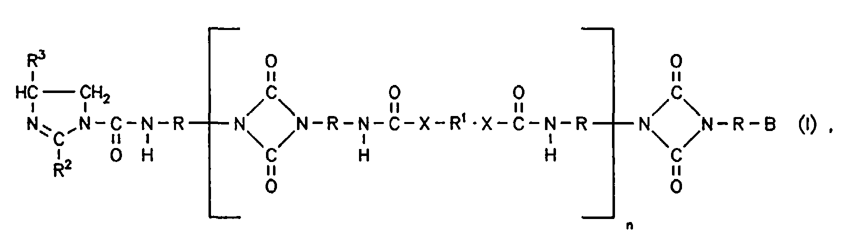 wobei
- X:: O, NR²,
- R:: der Kohlenwasserstoff-Rest des Isophorondiisocyanats: 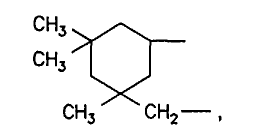
- R¹:: mit 0 - 3 CH₃-Gruppen substituierter (Cyclo)-alkylenrest mit 2 - 14 C-Atomen,
- R², R³:: gleiche oder verschiedene (Cyclo)alkylreste mit 1 - 10 C-Atomen oder Phenylreste,
- n:: 0 - 5,
- B:: 0 - 1 NCO-Gruppe und/oder 1 - 0 imidazolinblockierte NCO-Gruppe: 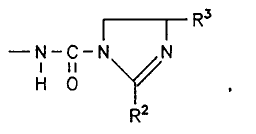 bedeuten.

## Beschreibung

Gegenstand der vorliegenden Erfindung sind cyclische Amidin- sowie Uretdiongruppen enthaltende Verbindungen, ein Verfahren zu ihrer Herstellung sowie deren Verwendung.

In der DE-OS 36 10 758 werden erstmals EP-Härter beschrieben, die die Härtung der Epoxidharze auf Bisphenol-A-Basis sowohl durch Polymerisation ihrer Epoxidgruppen (katalytische Reaktion) als auch durch Reaktion mit ihren OH-Gruppen (stöchiometrische Reaktion) bewirken. Die mit diesen Härtern hergestellten EP-Pulver zeichnen sich gegenüber den bekannten EP-Pulvern durch eine hervorragende Lösungsmittelbeständigkeit aus. Nachteilig bei den Pulvern der DE-OS 36 10 758 ist jedoch, daß sie nicht zum Verkleben von Metallen geeignet sind, da die damit verklebten Metallbleche bei erhöhten Temperaturen schlechte Zugscherfestigkeiten (DIN 53 283) aufweisen.

Überraschenderweise konnte dieser Nachteil beseitigt werden, indem man Verbindungen herstellt, die neben mit cyclischen Amidinen blockierte NCO-Gruppen noch Uretdiongruppen enthalten.

Gegenstand der vorliegenden Erfindung sind somit cyclische Amidin- sowie Uretdiongruppen enthaltende Verbindungen folgender Zusammensetzung: 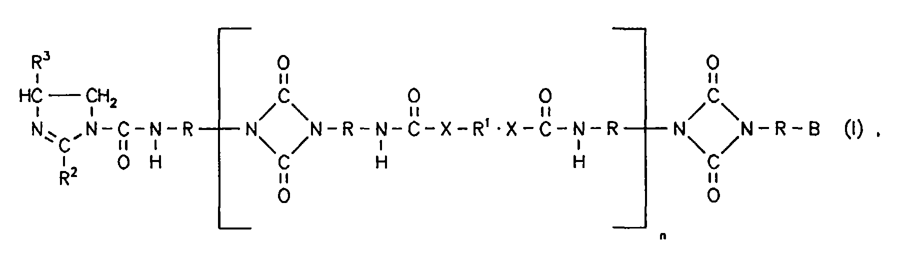 wobei
- X:: O, NR²,
- R:: der Kohlenwasserstoff-Rest des Isophorondiisocyanats: 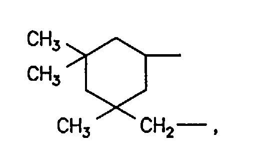
- R¹:: mit 0 - 3 CH₃-Gruppen substituierter (Cyclo)-alkylenrest mit 2 - 14 C-Atomen;
- R², R³:: gleiche oder verschiedene (Cyclo)alkylreste mit 1 - 10 C-Atomen bzw. Phenylreste,
- n:: 0 - 5,
- B:: 0 - 1 NCO-Gruppe und/oder 1 - 0 imidazolinblockierte NCO-Gruppe: 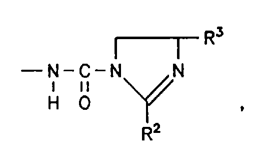
bedeuten.

Die erfindungsgemäßen Verbindungen zeichnen sich durch einen Gehalt an Uretdiongruppen von 1,5 - 2 mmol/g, an cyclischen Amidingruppen (Imidazoline) von 0,3 - 2 Gew.-%, an freien NCO-Gruppen von 0,2 - 7 Gew.-%, vorzugsweise 1 - 3 Gew.-%, aus. Ihr Schmelzpunkt liegt in einem Bereich von ca. 80 - 160 °C. Die erfindungsgemäßen Verbindungen eignen sich in hervorragender Weise zur Herstellung von lösungsmittelbeständigen Epoxidharzpulverbeschichtungen und zur Verklebung von Metallen mit verbesserter Zugscherfestigkeit bei erhöhten Temperaturen.

Ein weiterer Gegenstand der vorliegenden Erfindung ist ein Verfahren zur Herstellung der erfindungsgemäßen Verbindungen. Entsprechend dem erfindungsgemäßen Verfahren werden die Verbindungen (I) in zwei Stufen hergestellt, wobei in der 1. Stufe die Kettenverlängerung des Isophorondiisocyanat-Uretdions (abgekürzt: IPDI-Uretdion) mit einem Diol oder disekundären Diamin gemäß folgender Gleichung erfolgt: 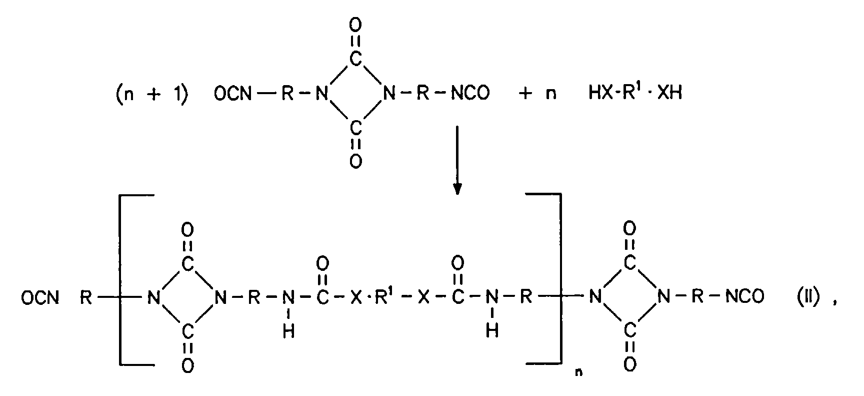 und x, R, R¹ und n die bereits angegebene Bedeutung haben.

Die Reaktion wird in inerten Lösungsmitteln, wie z. B. aromatischen Kohlenwasserstoffen, Estern oder Ketonen, durchgeführt; besonders vorteilhaft hat sich Aceton als Lösungsmittel erwiesen.

Das beim erfindungsgemäßen Verfahren eingesetzte IPDI-Uretdion wird in der DE-OS 30 30 513 beschrieben und hat einen NCO-Gehalt von 17 - 18 Gew.-%, der Monomergehalt ist < 0,7 Gew.-%; nach dem Erhitzen auf 180 °C (0,5 h) beträgt der NCO-Gehalt 37 - 37,6 Gew.-%.

Bei den für die Kettenverlängerung des IPDI-Uretdions in Frage kommenden Verbindungen handelt es sich einmal um Diole, wie sie z. B. in der DE-OS 27 38 270, S. 10 beschrieben werden, zum anderen um disekundäre Diamine, wie sie z. B. in bekannter Weise aus den entsprechenden di primären Diaminen durch Reaktion mit einer Carbonylverbindung, wie z. B. einem Keton oder Aldehyd, und anschließender Hydrierung gewonnen werden. Ein besonders einfaches Verfahren zur Herstellung der disekundären Verbindungen besteht in der Addition von Acrylsäureestern (CH₂=CH-COOR²) an die primären Aminogruppen der diprimären Diamine (H₂N-R¹-NH₂).

Bei der Kettenverlängerung des IPDI-Uretdions mit Diolen wird zu der acetonischen Lösung des IPDI-Uretdions das Diol, wie z. B. Ethylenglykol, Diethylenglykol, Butandiol, 3-Methylpentandiol-1,5, Hexandiol-1,6, Decandiol, Dodecandiol, 2,2,4(2,4,4)-Trimethylhexandiol-1,6, bei 60 °C zugegeben und so lange bei 60 °C weiter erhitzt, bis pro eingesetzte OH-Gruppe eine NCO-Gruppe reagiert hat. Zur Beschleunigung der Reaktion hat es sich als vorteilhaft erwiesen, 0,01 - 0,1 Gew.-% Dibutylzinndilaurat (DBTL) zuzugeben. Bei der Umsetzung des IPDI-Uretdions mit disekundären Diaminen wird zu der acetonischen IPDI-Uretdion-Lösung das Diamin bei Raumtemperatur so zudosiert, daß die Temperatur des Reaktionsgemisches 40 °C nicht übersteigt. Nach Beendigung der Diaminzugabe ist die Reaktion auch praktisch beendet. Die Zugabe eines Katalysators ist nicht erforderlich.

Zur Herstellung der erfindungsgemäßen Verbindungen wird nun das Reaktionsprodukt der 1. Stufe - die acetonische Lösung des kettenverlängerten IPDI-Uretdions - in einem 2. Reaktionsschritt mit dem cyclischen Amidin bei 60 °C umgesetzt. Das cyclische Amidin wird bei ca. 60 °C portionsweise zu der acetonischen Lösung des kettenverlängerten IPDI-Uretdions gegeben. Nach Beendigung der Amidin-Zugabe wird zur Vervollständigung der Reaktion noch ca. 1 h weiter erhitzt. Danach erfolgt die Entfernung des Acetons durch Destillation; zur Beseitigung der letzten Reste Aceton wird Vakuum angelegt. Als eine besonders vorteilhafte Isolierung des Reaktionsprodukts hat sich die Entfernung des Acetons in einem Filmextruder unter Vakuum erwiesen.

Nach dem erfindungsgemäßen Verfahren werden pro Mol kettenverlängertes IPDI-Uretdion ( 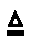 2 NCO-Äquivalente) 1 - 2 Mol cyclisches Amidin umgesetzt. Die zur Herstellung der erfindungsgemäßen Verbindungen geeigneten cyclischen Amidine werden in den DE-OSS 22 48 776 und 28 35 029 beschrieben. Besonders geeignet sind 2-Phenylimidazolin, 2-Phenyl-4-methylimidazolin sowie 2,4-Dimethylimidazolin.

Ein weiterer Gegenstand der vorliegenden Erfindung sind pulverförmige Überzugsmittel mit hoher Lagerstabilität und ausgezeichneter Lösungsmittelbeständigkeit auf der Basis von 1,2-Epoxidverbindungen mit mehr als einer 1,2-Epoxidgruppe und mehr als einer OH-Gruppe im Molekül, Härtungsmitteln und üblichen Lackzusätzen, dadurch gekennzeichnet, daß das Überzugsmittel als Härter folgende Verbindungen enthält: 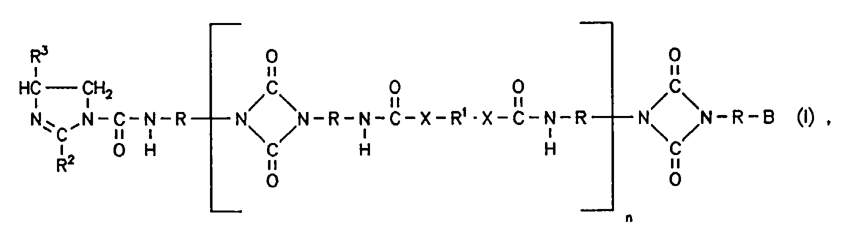 wobei X, R, R¹, R², R³, n und B die bereits im Anspruch 1 aufgeführten Bedeutungen haben, mit einem OH : NCO-Verhältnis von 1 : 0,25 bis 1 : 1, vorzugsweise 1 : 0,5, der Gehalt an cyclischem Amidin (in gebundener Form) 2 bis 8, vorzugsweise 3 bis 6 Gew.-%, bezogen auf die Summe Harz und Härter, beträgt und der Härter pro NCO-Äquivalent 0,5 bis 1 mol cyclisches Amidin enthält.

Die erfindungsgemäßen Härter (I) sind mit den OH-gruppenhaltigen EP-Harzen verträglich und liefern bei erhöhten Temperaturen homogene Schmelzen, die zur Herstellung von pulverförmigen Überzugsmitteln gut geeignet sind. Sie sind bei Raumtemperatur lagerstabil, die Aushärtezeiten liegen im Temperaturintervall von 160 - 200 °C innerhalb von 30 - 5 Minuten.

Zur Herstellung der erfindungsgemäßen pulverförmigen Überzugsmittel, die als Pulverlacke Verwendung finden sollen, sind natürlich nur solche Epoxidverbindungen geeignet, die mehr als eine OH-Gruppe im Molekül enthalten. Dies sind EP-Harze, die durch Reaktion von Bisphenol A und Epichlorhydrin im Molverhältnis n : (n+1) mit n : 2 - 7 erhalten werden. Besonders geeignet sind Epoxidharze mit einem EP-Äquivalentgewicht von ca. 900 und einem OH-Äquivalentgewicht von 300.

Die Herstellung der Pulverlacke erfolgt beispielsweise so, daß man die einzelnen Komponenten (EP-Harz, mit cyclischen Amidinen blockiertes kettenverlängertes IPDI-Uretdion und gegebenenfalls Zusätze, wie Verlaufsmittel, Pigmente, Füllstoffe, UV- und Oxidationsstabilisatoren) mahlt, mischt und bei 80 - 110 °C, vorzugsweise 90 - 100 °C, extrudiert. Nach dem Extrudieren wird abgekühlt und auf eine Korngröße kleiner 100 µm gemahlen. Bei der Herstellung des Bindemittelgemisches müssen die Komponenten so aufeinander abgestimmt werden, daß pro OH-Äquivalent des EP-Harzes 0,25 - 1, vorzugsweise 0,5, blockierte NCO-Gruppen des Härters kommen und dabei der cyclische Amidingehalt (in blockierter Form) 2 - 8, vorzugsweise 3 - 6 Gew.-%, bezogen auf die Summe Harz + Härter, beträgt. Der Härteranteil muß also so gewählt werden, daß sein cyclischer Amidingehalt zur katalytischen Härtung des EP-Harzes ausreicht (Polymerisation der Epoxidgruppen), ohne daß die OH-Gruppen reagieren und gleichzeitig eine Vernetzung des EP-Harzes durch Reaktion der OH-Gruppen des EP-Harzes mit den blockierten NCO-Gruppen des Härters, wobei allerdings die EP-Gruppen intakt bleiben, erreicht wird.

Die Applikation des Pulverlackes auf die zu überziehenden Substrate kann nach bekannten Methoden erfolgen, wie z. B. durch elektrostatisches Pulverspritzen oder Wirbelsintern. Anschließend werden die lackierten Gegenstände 5 - 30 Minuten im Temperaturbereich von 200 - 160 °C ausgehärtet. Zur Beschichtung mit den erfindungsgemäßen pulverförmigen Überzugsmitteln eignen sich alle Substrate, die die angegebenen Härtungsbedingungen vertragen, z. B. Metalle, Glas sowie Keramik. Die so hergestellten Pulverbeschichtungen zeichnen sich durch sehr gute lacktechnische Eigenschaften und eine hervorragende Lösungsmittelbeständigkeit gegenüber aggressiven Lösungsmittel, wie z. B. Methylisobutylketon, aus.

Ein weiterer Gegenstand der vorliegenden Erfindung betrifft die Verwendung der erfindungsgemäßen Verbindungen zur Herstellung von pulverförmigen, einkomponentigen Metallklebstoffen.

Das zur Verklebung von Metallen in Frage kommende Harz/Härter-Gemisch ist mit den pulverförmigen Überzugsmitteln identisch, d. h. gleiche Zusammensetzung, Herstellung wie auch Applikation, wobei es bei der Verklebung von Metallen sogar ausreichend ist, das Pulver auf die Bleche aufzusieben. Nachdem die gereinigte Oberfläche des einen Metalls mit dem erfindungsgemäßen Harz/Härter-Gemisch beschichtet wurde, wird es mit dem zu verklebenden anderen Metall mit Hilfe einer Schraubzwinge fixiert. Die Aushärtung erfolgt wie bei den pulverbeschichteten Substraten bei 160 - 200 °C innerhalb von 30 - 5 Minuten.

Die so hergestellten Metallverklebungen unterscheiden sich deutlich von den z. Z. auf dem Markt erhältlichen 1K-Metallklebstoffen auf EP-Basis hinsichtlich ihrer Festigkeit (Zugscherfestigkeit nach DIN 53 283) bei höheren Temperaturen.

Die auf dem Markt befindlichen pulverförmigen Metallklebstoffe auf Basis von EP-Harzen bestehen aus einem (festen) EP-Harz, das mit Dicyandiamid gehärtet wird. Die damit hergestellten Metallverklebungen zeigen bei Raumtemperatur ausgezeichnete Zugscherfestigkeiten, die jedoch mit zunehmender Temperatur stark abnehmen und bei 150 °C nahezu Null sind, d. h. daß bei 150 °C die Verklebung versagt, dagegen zeigen Verklebungen mit dem erfindungsgemäßen Harz/Härter-Gemisch bei 150 °C noch Zugscherfestigkeiten von ca. 10 N/mm² auf.

### A) Allgemeine Herstellungsvorschrift für die erfindungsgemäßen Verbindungen

Zu der acetonischen Lösung des IPDI-Uretdions (ca. 50 % Aceton, bezogen auf die Summe IPDI-Uretdion + Kettenverlängerungsmittel + cyclisches Amidin), die 0,05 Gew.-% Dibutylzinndilaurat enthält, wird bei 60 °C das Diol innerhalb von ca. 1 h unter intensiver Rührung zudosiert und so lange bei 60 °C weiter erhitzt, bis pro eingesetztes OH-Äquivalent 1 NCO-Äquivalent umgesetzt wurde. Danach wird das cyclische Amidin portionsweise zugegeben. Nach erfolgter Amidin-Zugabe wird noch ca. 1 h bei 60 °C weiter erhitzt. Anschließend wird das Aceton abdestilliert; zur Entfernung der letzten Reste Aceton wird Vakuum angelegt. Wird anstelle des Diols ein disekundäres Diamin zur Kettenverlängerung des IPDI-Uretdions eingesetzt, erfolgt die Umsetzung bei Raumtemperatur und ohne DBTL.

Das zur Kettenverlängerung eingesetzte IPDI-Uretdion wurde entsprechend den im Beispiel 2 der DE-OS 30 30 513 beschriebenen Reaktionsbedingungen hergestellt. Der NCO-Gehalt des IPDI-Uretdions betrug 17,3 %; beim Erhitzen auf 180 °C (0,5 h) wurde ein NCO-Gehalt von 37 % gefunden.

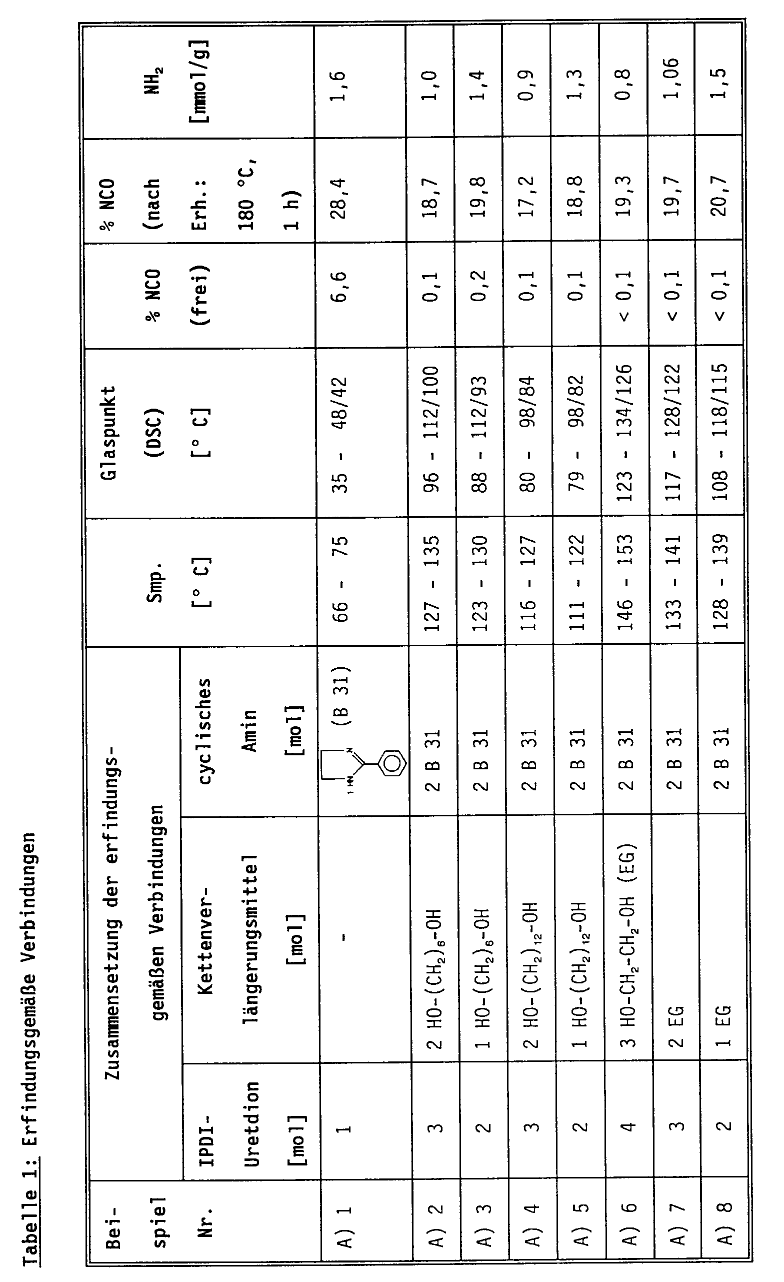

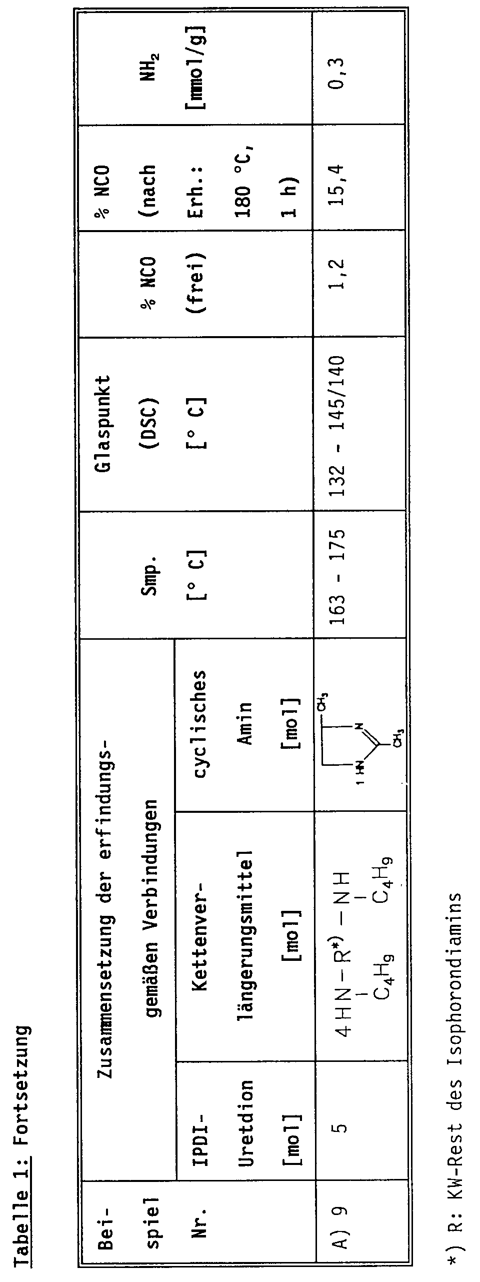

### B) Epoxidharz-Pulverlacke

### Allgemeine Herstellungsvorschrift

Die gemahlenen Produkte - Härter, Epoxidharz und Verlaufsmittel-Masterbatch - wurden mit dem Weißpigment zunächst in einem Kollergang trocken vermischt und anschließend im Extruder bei 80 - 120 °C homogenisiert. Nach dem Erkalten wurde das Extrudat gebrochen und auf einer Stiftmühle auf eine Korngröße < 100 µm gemahlen. Das so hergestellte Pulver wurde mit einer elektrostatischen Pulverspritzanlage bei 60 KV auf entfettete, gegebenenfalls vorbehandelte Eisenbleche (1 mm Stärke) appliziert und in einem Labor-Umlufttrockenschrank eingebrannt.

### Verlaufsmittel-Masterbatch

Es werden 10 Gew.-% des Verlaufsmittels - ein handelsübliches Acrylatoligomeres - im Epoxidharz in der Schmelze homogenisiert und nach dein Erstarren zerkleinert.

### Epoxidharz

In den nachstehenden Epoxidharz-Lackbeispielen wurde ein festes Epoxidharz des Typs Diglycidylether des Bisphenol A und Epichlorhydrin verwendet, welches nach Angaben des Herstellers ein Epoxid-Äquivalentgewicht von 900 - 1000, einen Epoxidwert von 0,10 - 0,11, einen Hydroxylwert von 0,34 und einen Schmelzpunkt von 96 - 104 °C aufweist.

### Zusammensetzung des EP-Pulverlackes

Die in der Tabelle 2 aufgeführten Pulverlacke enthalten 40 Gew.-T. TiO₂, 0,5 Gew.-T. Verlaufsmittel, 59,5 Gew.T. Bindemittel, wobei das OH : NCO-Äquivalenzverhältnis von Harz zu Härter in der Regel 2 : 1 beträgt.

Die Abkürzungen in der folgenden Tabelle bedeuten:
- SD:: Schichtdicke in µm
- HK:: Härte nach König in sec (DIN 53 157)
- ET:: Tiefung nach Erichsen in mm (DIN 53 156)
- GG 60 °∢:: Glanzgrad nach Gardner (ASTM-D 523)
- Imp. rev.:: Impact reverse in g·m
- GS:: Gitterschnittprüfung (DIN 53 151)
- MEK (Methylethylketon)-Festigkeit:: Anzahl Schübe mit MEK-getränktem Wattebausch unter 1-kg-Belastung bis zum Angriff (matter Oberfläche)

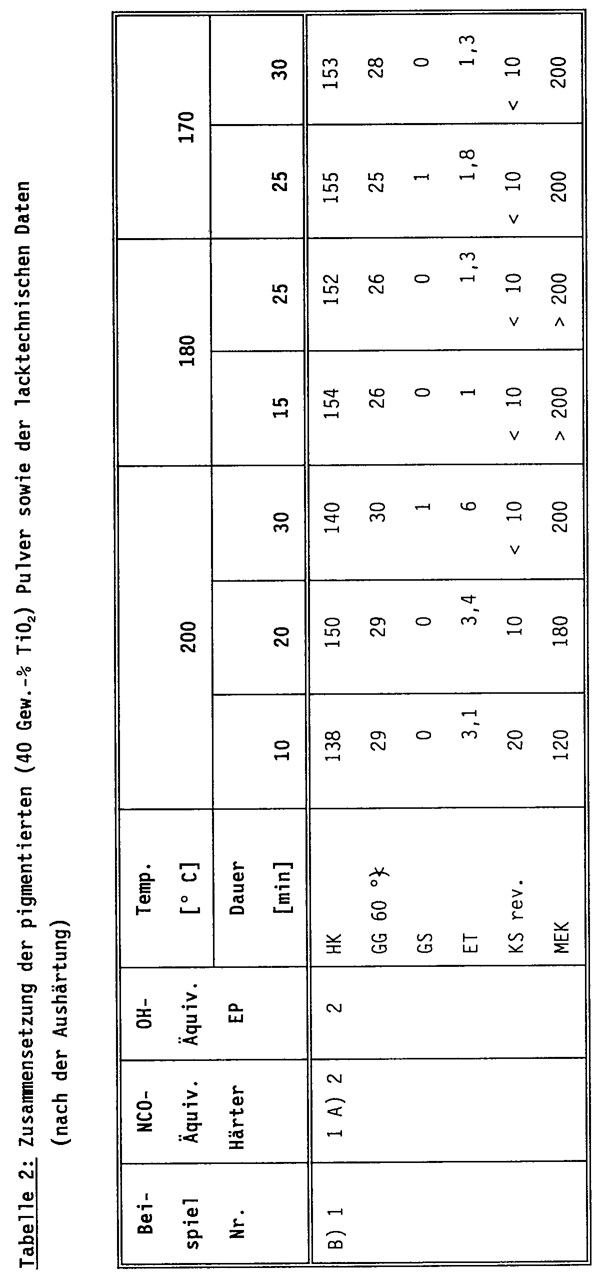

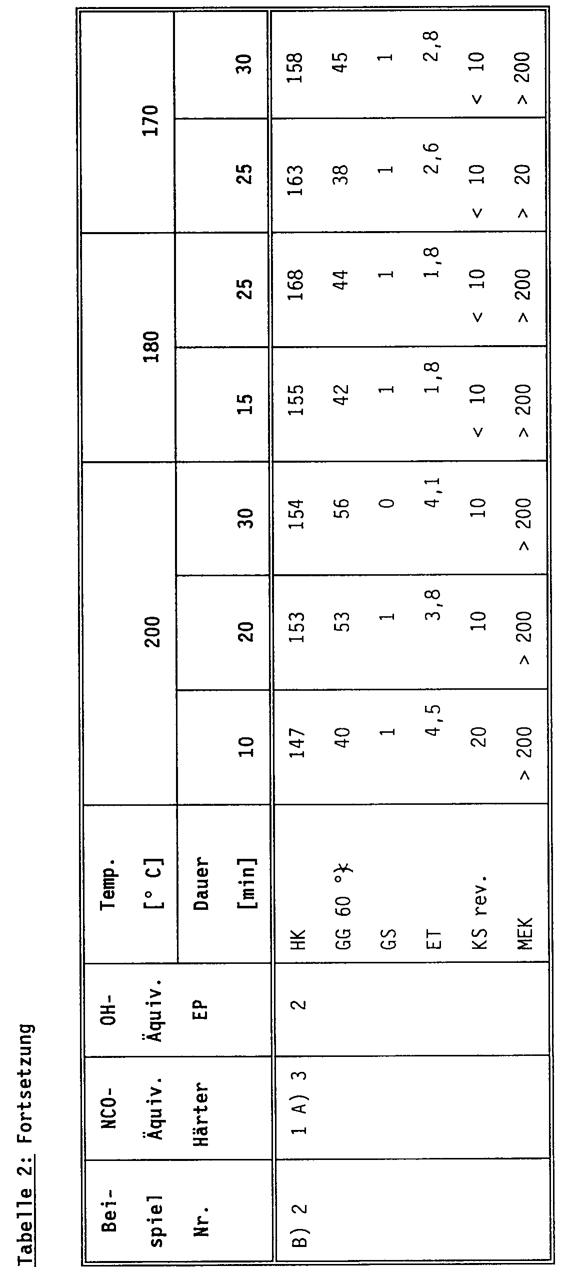

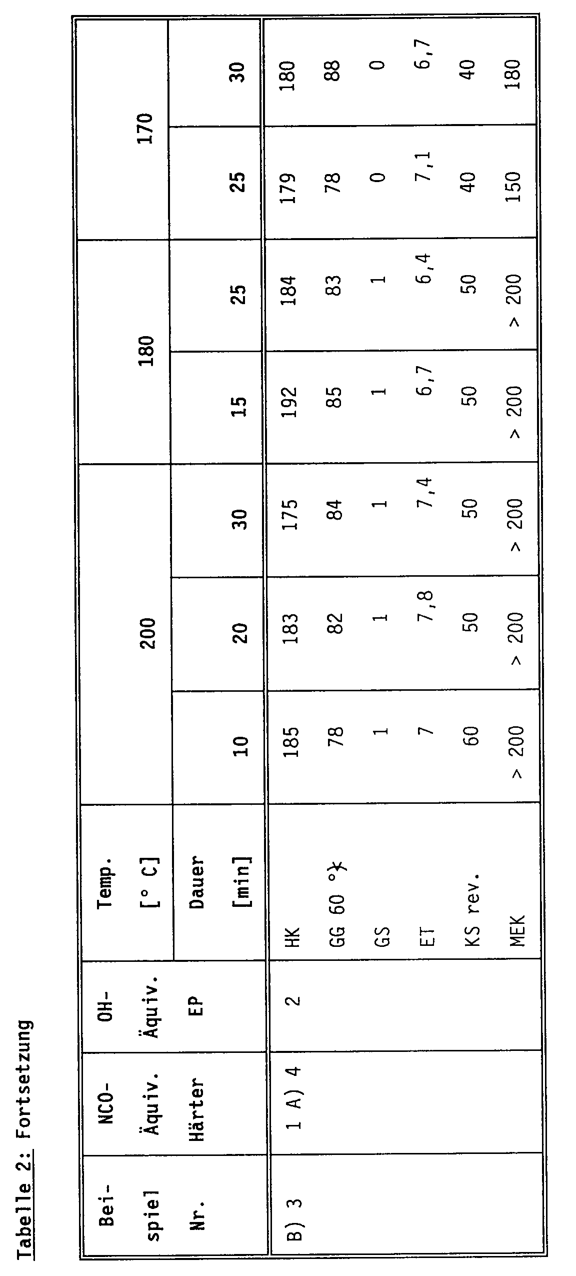

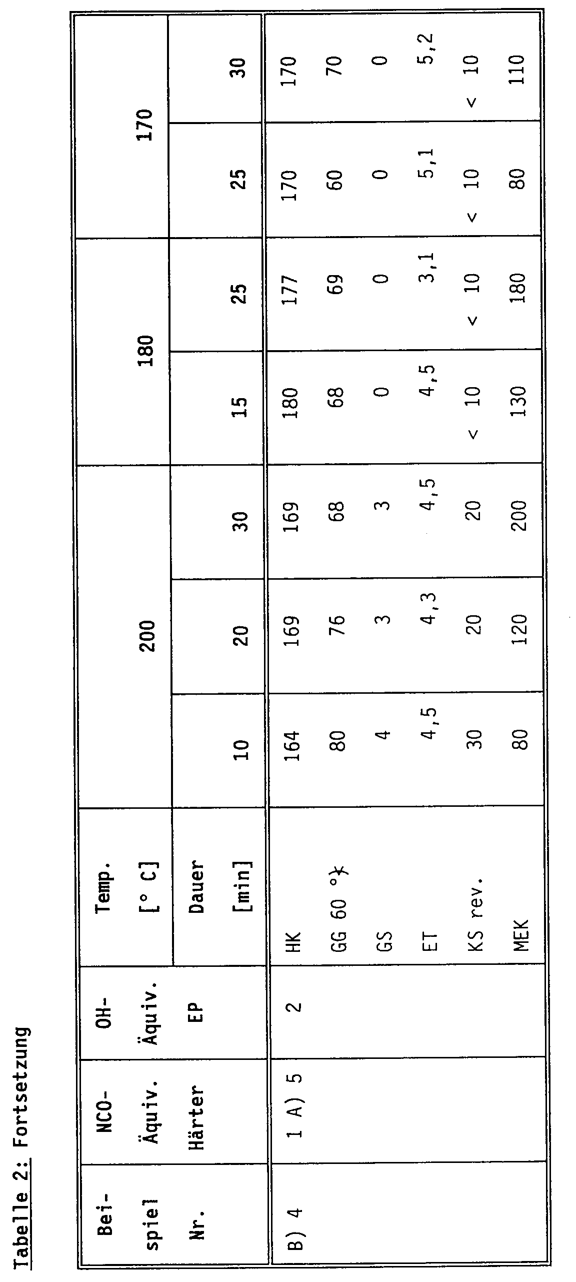

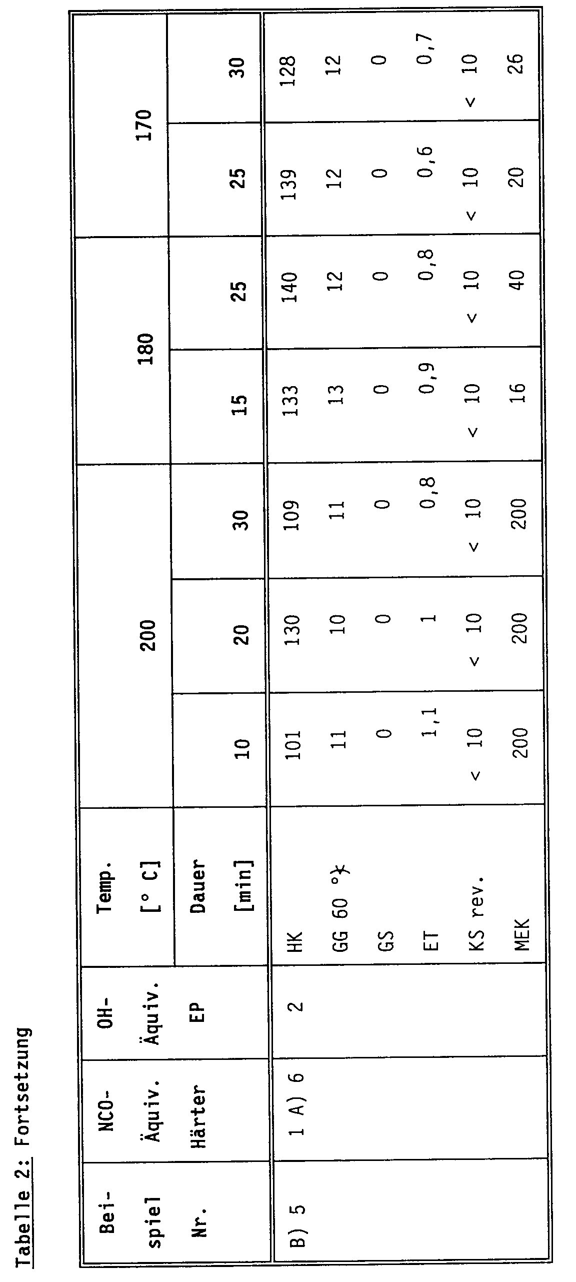

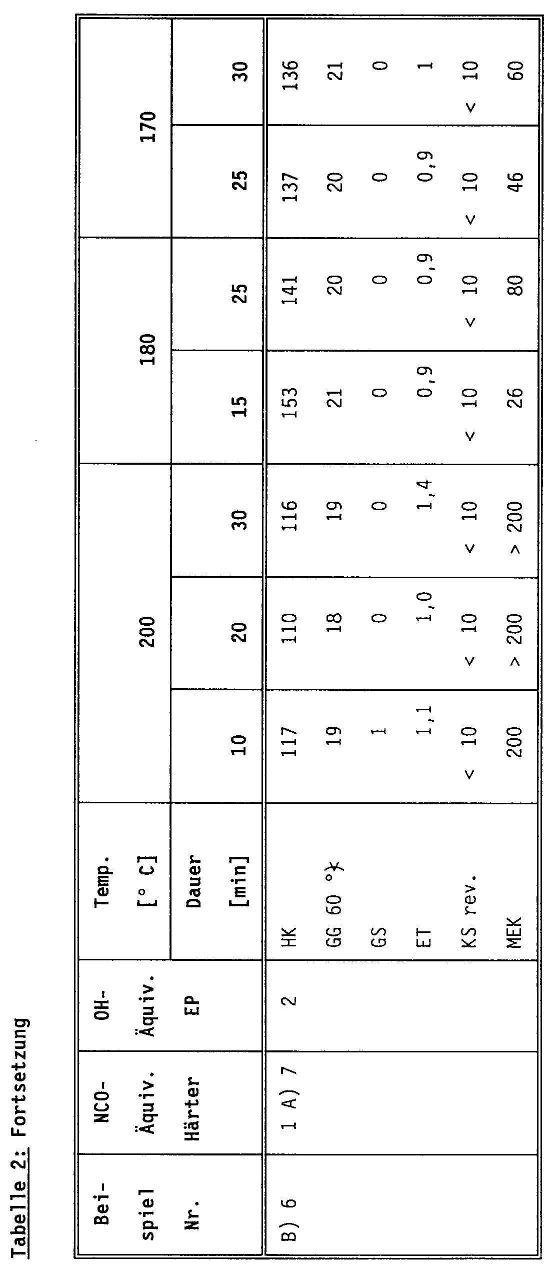

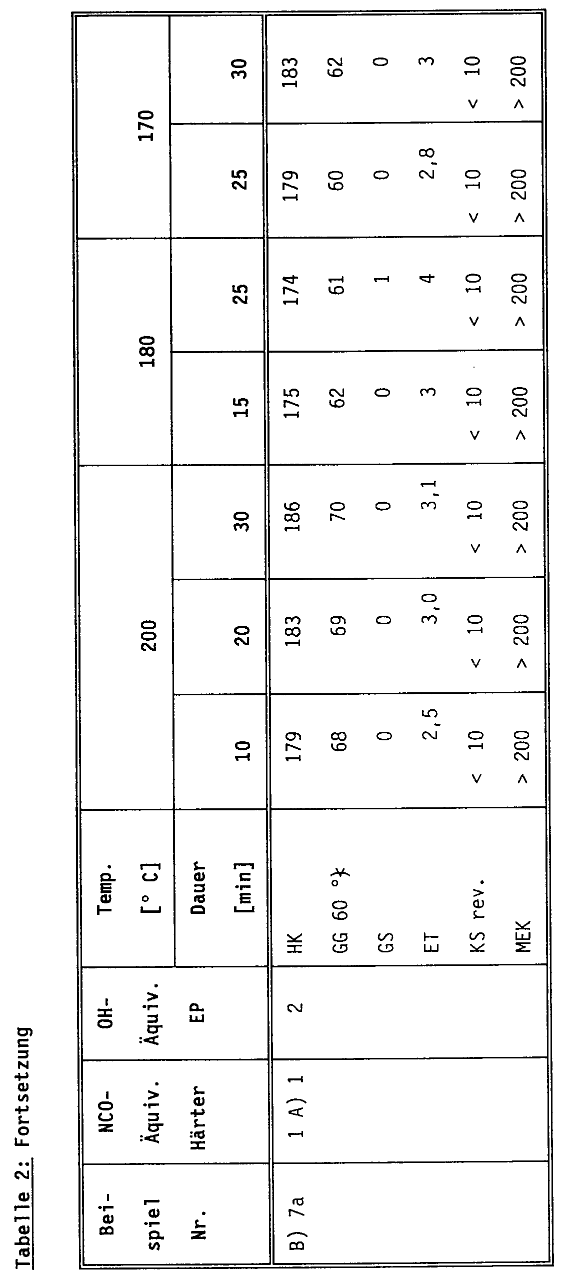

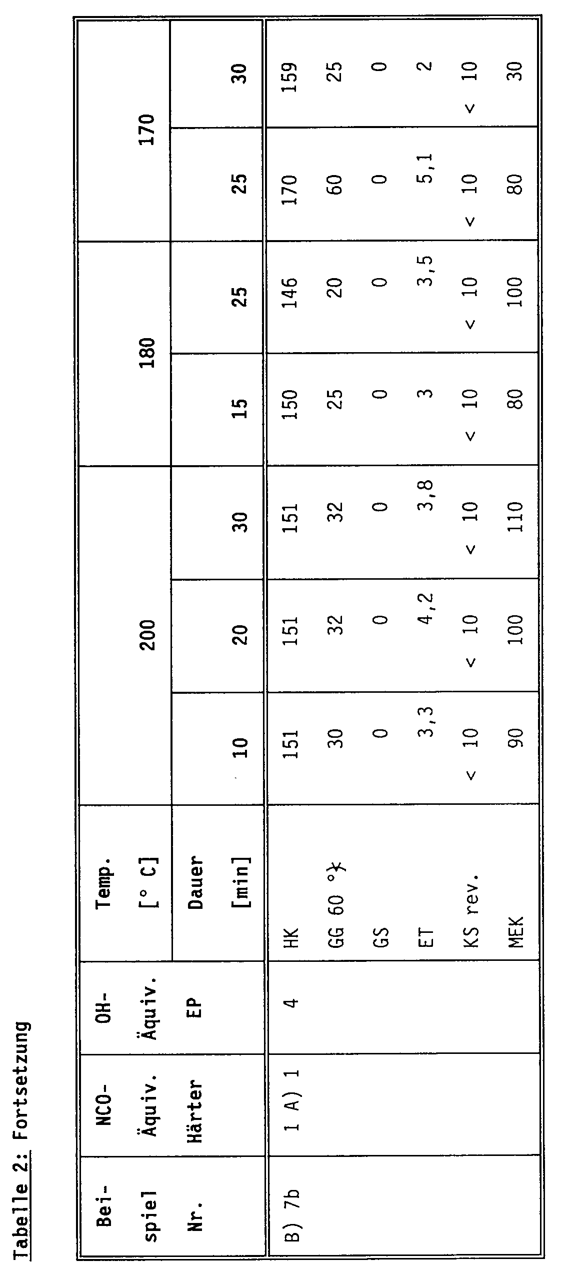

### C) Verwendung der erfindungsgemäßen Verbindungen zur Herstellung von pulverförmigen 1K-Klebstoffen

Die erfindungsgemäßen Härter und das EP-Harz mit einem EP-Wert von 0,1 werden bei 100 °C 5 min in einem Plastographen intensiv geknetet. Nach dem Abkühlen wird das Produkt gemahlen und auf die mit ScotchBrite gereinigten Stahlbleche (1,5 mm dick) gesiebt und nach DIN 53 283 verklebt. Die Zugscherfestigkeiten dieser Stahlverklebungen (nach Härtung bei 200 bzw. 180 °C) sind in der nachfolgenden Tabelle aufgelistet.

**Tabelle 3**

| **Metallverklebungen (DIN 53 283) mit den erfindungsgemäßen Härter/EP-Gemischen** | | | | | | | | |
|---|---|---|---|---|---|---|---|---|
| **Beispiel Nr.** | **Klebstoffzusammensetzung** | | **Härtung** | | **Zugscherfestigkeit (DIN 53 283) [N/mm**^{**2**}**]** | | | |
| | **NCO-Äquiv. Härter** | **OH- Äquiv. EP** | **Temperatur [° C]** | **Zeit [min]** | **Raumtemperatur** | **100 °C** | **130 °C** | **150 °C** |
| C) 1 | 1 A) 7 | 2 | 180 | 30 | 20 | 16 | 10 | 9 |
| C) 2 | 1 A) 7 | 1 | 180 | 30 | 21 | 19 | 9 | 9 |
| C) 3 | 1 A) 8 | 2 | 180 | 30 | 20 | 17 | 8 | 7 |
| C) 4 | 1 A) 8 | 1 | 180 | 30 | 18 | 19 | 16 | 15 |
| C) 5 | 1 A) 4 | 2 | 180 | 30 | 21 | 19 | 7 | 6 |
| | | | 200 | 15 | 20 | 18 | 9 | 7 |
| C) 6 | 1 A) 4 | 1 | 180 | 30 | 17 | 13 | 13 | 12 |
| | | | 200 | 15 | 18 | 14 | 14 | 15 |
| C) 7 | 1 A) 5 | 2 | 180 | 30 | 21 | 18 | 17 | 14 |
| | | | 200 | 15 | 20 | 18 | 16 | 16 |

| Vergleichsbeispiel | | | | | | | | |
|---|---|---|---|---|---|---|---|---|
| AT 1 (Ciba) | | | 200 | 30 | 29 | 14 | 3 | 1 |

## Patentansprüche

1. Cyclische Amidin- sowie Uretdiongruppen enthaltende Verbindungen folgender Zusammensetzung: 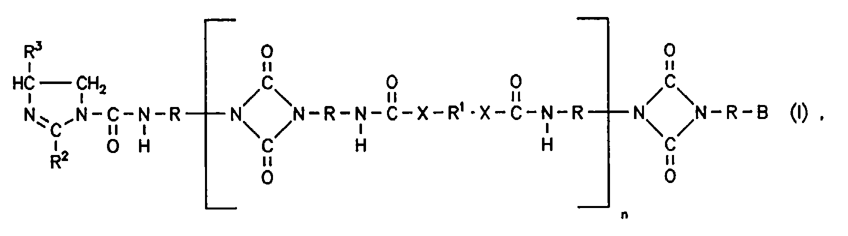 wobei
X: O, NR²,
R: der Kohlenwasserstoff-Rest des Isophorondiisocyanats: 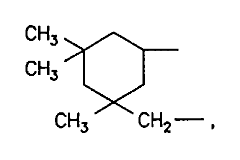
R¹: mit 0 - 3 CH₃-Gruppen substituierter (Cyclo)-alkylenrest mit 2 - 14 C-Atomen,
R², R³: gleiche oder verschiedene (Cyclo)alkylreste mit 1 - 10 C-Atomen oder Phenylreste,
n: 0 - 5,
B: 0 - 1 NCO-Gruppe und/oder 1 - 0 imidazolinblockierte NCO-Gruppe: 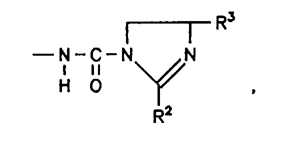
bedeuten.

2. Verbindungen nach Anspruch 1,
dadurch gekennzeichnet,
daß Reste von 2-Phenylimidazolin, 2-Phenyl-4-methylimidazolin oder 2,4-Dimethylimidazolin enthalten sind.

3. Verfahren zur Herstellung der erfindungsgemäßen Verbindungen gemäß den Ansprüchen 1 bis 2,
dadurch gekennzeichnet,
daß in einer 1. Stufe Isophorondiisocyanat-Uretdion mit Diolen oder disekundären Diaminen entsprechend folgender Gleichung: 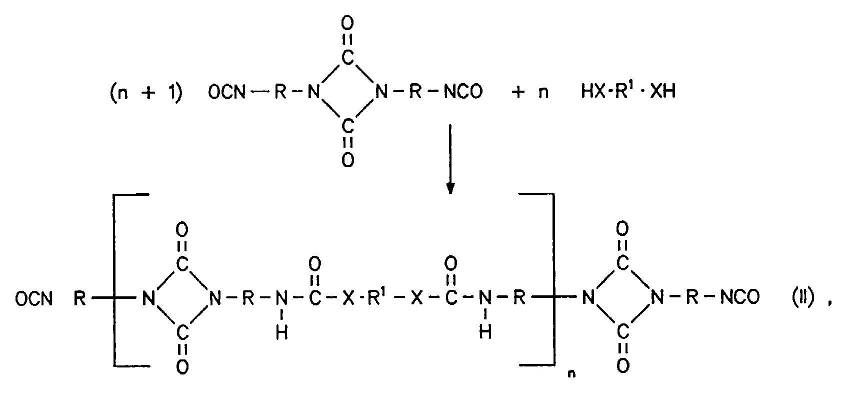 in inerten Lösungsmitteln umgesetzt wird und in der 2. Stufe das kettenverlängerte Isophorondiisocyanat-Uretdion (II) teilweise oder ganz mit cyclischen Amidinen der allgemeinen Formel: 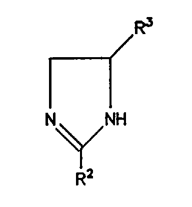 umsetzt, wobei X, R, R¹, R², R³ und n die im Anspruch 1 angegebene Bedeutung haben.

4. Verfahren nach Anspruch 3,
dadurch gekennzeichnet,
daß als Lösemittel Aceton eingesetzt wird.

5. Pulverförmige Überzugsmittel mit guter Lagerstabilität und ausgezeichneter Lösungsmittelbeständigkeit auf der Basis von 1,2-Epoxidverbindungen mit mehr als einer 1,2-Epoxidgruppe und mehr als einer OH-Gruppe im Molekül, Härtungsmitteln und üblichen Lackzusätzen,
dadurch gekennzeichnet,
daß das Überzugsmittel als Härter folgende Verbindungen enthält: 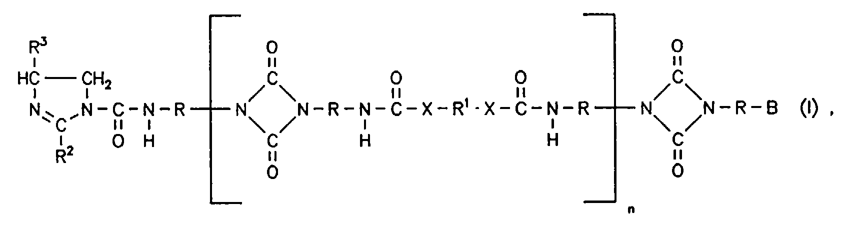 wobei X, R, R¹, R², R³, n und B die bereits im Anspruch 1 aufgeführten Bedeutungen haben, mit einem OH : NCO-Verhältnis von 1 : 0,25 bis 1 : 1, der Gehalt an cyclischem Amidin (in gebundener Form) 2 bis 8 Gew.-%, bezogen auf die Summe Harz und Härter, beträgt und der Härter pro NCO-Äquivalent 0,5 bis 1 mol cyclisches Amidin enthält.

6. Pulverförmige Überzugsmittel nach Anspruch 5,
dadurch gekennzeichnet,
daß das OH : NCO-Verhältnis 1 : 0,5 beträgt.

7. Pulverförmige Überzugsmittel nach den Ansprüchen 5 bis 6,
dadurch gekennzeichnet,
daß der Gehalt an cyclischem Amidin 3 bis 6 Gew.-% beträgt.

8. Verwendung der pulverförmigen Überzugsmittel zur Herstellung von pulverförmigen 1K-Metallklebstoffen.
